Europäisches Patentamt

European Patent Office

Office européen des brevets

(19)

(11) Veröffentlichungsnummer: **0 509 318 A1**

## (12) EUROPÄISCHE PATENTANMELDUNG

(21) Anmeldenummer: **92105602.4**

(22) Anmeldetag: **01.04.92**

(51) Int. Cl.5: **A61N 1/16**

(30) Priorität: **16.04.91 CH 1126/91**

(43) Veröffentlichungstag der Anmeldung:
**21.10.92 Patentblatt 92/43**

(84) Benannte Vertragsstaaten:
**AT BE DE ES FR GB IT NL SE**

(71) Anmelder: **Gantenbein, Walter**
**Hus Sunneschy**
**CH-9472 Grabs(CH)**

(72) Erfinder: **Gantenbein, Walter**
**Hus Sunneschy**
**CH-9472 Grabs(CH)**

(74) Vertreter: **Büchel, Kurt F., Dr.**
**Bergstrasse 297**
**FL-9495 Triesen(LI)**

(54) **Zusammenklappbares Gestell aus vier an einem Ende miteinander verbundenen Stangen.**

(57) Die Erfindung betrifft ein zusammenklappbares Pyramiden-Gestell (2) aus vier Winkelprofilen (1), die an jeweils einem ihrer Enden miteinander verbunden sind. Die Winkelprofile (1) stützen sich über eine quadratische Grundfläche, mit der sie einen Winkel ($\alpha$) von vorzugsweise 56,5° einschliessen. Ein solches Gestell erreicht ähnliche Auswirkungen auf biologische Vorgänge wie Pyramidenaufbauten aus vollwandigem Kupferblech.

Fig 1

Die Erfindung betrifft ein zusammenklappbares Gestell nach dem Oberbegriff des Anspruches 1.

Durch verschiedene Untersuchungen und Studien, wie sie beispielsweise in dem Buch "Praxis der Pyramidenenergie" von Rudi Ph. Weilmünster 1988 vorgestellt wurden, wurde nachgewiesen, dass Pyramiden aus Metall bestimmte, zunächst noch nicht weiter erklärbare Einflüsse auf biologische Vorgänge ausüben können. Üblich ist es dabei, Pyramiden aus Kupferblech zu falten oder aus dreieckförmigen Blechen zusammenzustellen.

Es wurde in neueren Untersuchungen erkannt, dass die vollwandigen Ausführungen für das Erzielen der verschiedenen Wirkungen gar nicht erforderlich sind, sondern dass vielmehr auch pyramidenförmige Gestelle, die lediglich aus vier zusammengestellten Stangen aufgebaut sind, die gleichen Effekte erzielen können.

Dies ist an sich ein enormer Vorteil, da das Bearbeiten und Transportieren von Kupferplatten aufwendig und wegen der Verletzungsgefahr zum Teil auch gefährlich ist. Pyramidenförmige Gestelle sind hingegen in nahezu beliebiger Grösse herstellbar und leicht zu transportieren.

Besonders für Anwendungen im Gartenbau, aber auch in Lagerräumen und in Räumen zu Meditationszwecken eignen sich daher aus Stangen aufgebaute pyramidenähnliche Gestelle optimal. Darüber hinaus können sogar ganze Betten usw. ins Innere des Gestells verbracht werden. Darüber hinaus kann das Gestell selbst auch Trägerfunktion übernehmen. So kann ein Bett z.B. an den vier Winkelprofilen befestigt sein oder an einem Haken im Spitz hängend montiert werden. Die Erfindung beschäftigt sich mit dem Verbessern solch zusammenklappbarer Gestelle im Hinblick auf eine Optimierung der Wirkung des pyramidenförmigen Gestelles.

Im Zuge von Untersuchungen wurde überraschend festgestellt, dass die Form der pyramidenförmig zusammengestellten Stangen von erheblicher Bedeutung ist, ebenso wie der sich durch die Stangen ergebende Pyramidenspitz. Es wurde erkannt, dass z.B. zylinderförmige Rohre wesentlich weniger Effekte erzielen, als kantige Winkelprofile. Dementsprechend wurde erfindungsgemäss ein neuartiges Gestell entwickelt, das die kennzeichnenden Merkmale des Anspruches 1 aufweist. Weitere Ausführungsformen und Verbesserungen des Erfindungsgedankens sind in den Kennzeichen der abhängigen Ansprüche beschrieben.

Die Wirkung solcher erfindungsgemässer Gestelle auf biologische Vorgänge, wie Alterung von Lebensmitteln, Wachstum von Pflanzen und Geschmackserhaltung von Getränken, ist unvergleichlich besser als bei Gestellen aus anders geformten Stangen. Die Wirkung kommt durchaus an jene von vollwandigen Pyramiden heran. Diesen gegenüber bieten erfindungsgemässe Gestelle jedoch den Vorteil einer leichten Transportierbarkeit und eines geringeren Materialaufwandes. Beim Transport bilden sie ein stabiles Rohr. Erfindungsgemässe Gestelle sind formstabil und in nahezu beliebiger Höhe herstellbar.

Als optimales Material bieten sich Winkelprofile aus Kupfer oder einer Kupferlegierung an. Es gilt dabei die Theorie, dass die diamagnetischen Eigenschaften eines Materials für die optimale Funktionsweise ausschlaggebend sind. Es bieten sich als Alternative zum Kupfer daher auch verschiedene andere Materialien, wie z.B. Bismuth oder neue keramische Supraleiter an. Diese Materialien sind - wenngleich durch die Erfindung auch umfasst - nicht als bevorzugt anzusehen, da sie sehr teuer sind.

Es gibt verschiedene Möglichkeiten für unterschiedliche Anstellwinkel von erfindungsgemässen Gestellen, wobei der richtige Anstellwinkel mit Vorzug dadurch erreichbar ist, dass die Stangen an ihrem freien Ende durch Abstandhalter - z.B. Metallketten - verbindbar sind.

Alternativ dazu und mit grösserer Präzision können auch Grundplatten aus Kupfer vorgesehen sein, die die freien Enden des Gestells an dafür vorgesehenen Stellen aufnehmen.

Es ist bekannt, dass eine bzw. zwei der Basiskanten einer Pyramide zur Erzielung der Wirkung auf die biologischen Vorgänge eine Nord-Süd-Ausrichtung benötigen. Gemäss einer Weiterentwicklung der Erfindung kann diese Nord-Süd-Orientierung am einfachsten dadurch erzielt werden, dass mit einer der Stangen ein Kompass verbunden ist, der von oben bzw. seitlich gut ablesbar ist und so das Justieren des Gestelles ermöglicht. Mit Vorzug ist der Kompass an der Innenseite der Schenkel eines Winkelprofils befestigt und zwar an jenem Winkelprofil, das beim Zusammenklappen des Gestells innen liegt. Der Kompass ist dadurch auch beim Transport bestens geschützt.

Besonders gute Wirkungen wurden mit einem Gestell erzielt, bei dem die Länge eines Winkelprofils etwa um den Faktor 1,28 grösser war als der Abstand zwischen zwei freien Enden des Gestells bei voll aufgespreizten Stangen. Allerdings funktionieren auch Gestelle mit geringfügigen Abweichungen von diesem Mass aktivierend. Bei überzogenen (vollwandigen) Pyramiden werden eher kleinere Faktoren empfohlen.

Eine wichtige Anwendung der Erfindung ist im Anspruch 10 beschrieben. Metallfolien können im Inneren des Pyramidengestelles biologisch aktiviert (aufgeladen) werden. Versuche haben gezeigt, dass Aluminiumfolien, die ein paar Tage ins Innere des Gestells gelegt wurden, heilende Wirkung auf kranke Körperstellen von Versuchspersonen hatten. Personen, die längere Zeit auf Sitz- oder Liegemöbeln verbringen, erreichen meditationswirksame Zustände.

Weitere Details und Ausführungsmerkmale der Erfindung ergeben sich aus der Zeichnung und den Beschreibungen der Figuren.

Es zeigen dabei die

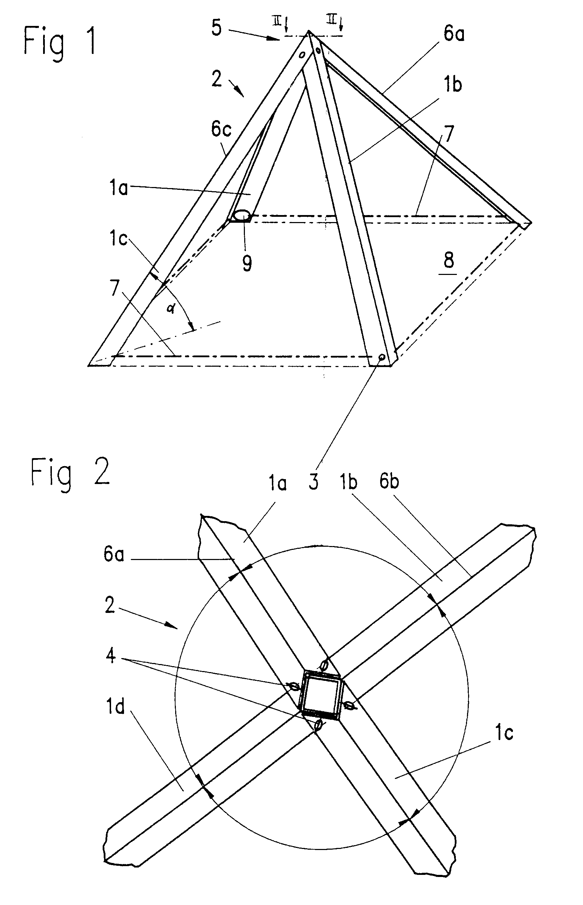

Fig.1    die Schrägsicht auf ein aufgespreiztes Gestell und

Fig.2    die Draufsicht auf ein erfindungsgemässes Gestell mit einem Schnitt entlang der Linie II - II in Fig.1.

Die Figuren werden zusammenhängend be- schrieben. Gleiche Bezugszeichen betreffen glei- che Bauteile, gleiche Bezugszeichen mit unterschiedlichen Indizes betreffen ähnliche Bauteile.

Das zusammenklappbare Gestell 2 besteht aus vier Winkelprofilen 1a - 1d, die an ihren jeweiligen Berührungsstellen miteinander durch schematisch angeordnete Schrauben 4 verschraubt sind. Die Winkelprofile 1 stützen sich über einer quadratischen Grundfläche 8, so dass sie von dem jeweils benachbarten Winkelprofil 1 um den Winkel $\beta$ ver- setzt sind. Die Winkelprofile sind vorzugsweise scharfkantig, wobei die Kante nach aussen gerichtet ist, und sind an dem Ende, an dem sie zusammengefügt sind, so zugeschnitten, dass sie im aufgespreizten Zustand eine ziemlich scharfe Spitze ergeben. Als Material kommt hauptsächlich diamagnetisches Material, wie z.B. Kupfer oder dergleichen in Frage. Gegen zu weites Aufspreizen sichern vier Ketten 7, die zwischen je zwei benachbarten Winkelprofilen 1 gespannt sind.

Das Gestell 2 muss mit einer Basiskante, das heisst - wenn vorhanden - mit einer der Ketten 7 Nord-Süd orientiert sein. Der Winkel $\alpha$ ist für opti- male Wirkung abhängig vom Breitengrad des Aufstellungsortes der Pyramide. Für den 43. Breitengrad ist es zweckdienlich, wenn die Länge eines Winkelprofils 1 etwa 1,28 mal grösser ist, als die Kantenlänge des Quadrates 8, über das sich das aufgespreizte Gestell 2 stützt. Damit ist $\alpha$ etwa 56,5°.

Für besondere Anwendungsfälle kann noch eine separate Erdungsklemme 3 vorgesehen sein, mit der das Gestell auf elektrisches Erdpotential gelegt werden kann.

Um gegebenenfalls die Anstellwinkel $\alpha$ variie- ren zu können, ist eine Variation der Länge der Kette möglich. Es können aber auch längs der Winkelprofile mehrere Bohrungen für das Einhängen der Kette vorgesehen sein, so dass sich da- durch der Anstellwinkel $\alpha$ variieren lässt.

Beim Zusammenbau des Gestells geht man am besten so vor, dass man zuerst den richtigen Winkel der Winkelprofile ermittelt, anschliessend je zwei solcher Profile miteinander verbindet und diese beiden Winkelprofilpaare dann zum Gestell zusammenbaut. Vorzugsweise sollen alle verwendeten Materialien von gleicher Konsistenz sein.

Die Erfindung ist durch das dargestellte Beispiel nicht eingeschränkt. Im Rahmen der Ansprüche sind noch verschiedene Variationen denkbar. So können zum Beispiel anstelle von Schrauben auch Niete oder Bolzen vorgesehen sein, die z.B. kreuzweise durch Bohrungen im Spitz 5 gesteckt sind. Ausserdem ist es denkbar, über den Spitz 5 noch zusätzlich eine pyramidenförmige Kappe zu stülpen, die die geometrische Form noch verbessert. Innerhalb des Pyramidengestells können noch weitere Pyramiden und/oder Gestelle angeordnet werden. Zum Schutz vor Oxidation können die Winkelprofile auch lackiert oder galvanisch oberflächenbehandelt - z.B. auch goldplattiert - sein.

Für besondere Anwendungsfälle ist es auch denkbar, das gesamte Gestell durch eine Folie oder auch durch Stoff, Papier, Pappe oder dgl. abzudecken, so dass die freien Öffnungen zwischen den Winkelprofilen abgedeckt sind.

**Patentansprüche**

1.    Zusammenklappbares Pyramiden-Gestell (2) aus vier an einem Ende unmittelbar durch Ver- bindungsmittel miteinander verbindbaren Stan- gen, dadurch gekennzeichnet, dass die Stangen aus einem Winkelprofil (1) gebildet sind, dessen Winkel in Abhängigkeit von dem gewünschten Anstellwinkel der Stangen so ausgebildet ist, dass sich je eine Seitenfläche des Winkelprofils (1) an eine Seitenfläche eines benachbarten Winkelprofils (1) flächig anschmiegt, wobei die sich jeweils anschmiegen- den Seitenflächen relativ zueinander ver- schwenkbar sind, wobei die Aufstellung der Stangen in den Anstellwinkel ($\alpha$) die Ausbil- dung eines pyramidenförmigen Spitzes (5) ermöglicht.

2.    Gestell nach Anspruch 1, dadurch gekennzeichnet, dass die Winkelprofile (1) aus einem Material mit hohen diamagnetischen Eigenschaften, z.B. insbesondere einem kupferhaltigen Material, aufgebaut sind.

3.    Gestell nach einem der vorhergehenden Ansprüche, dadurch gekennzeichnet, dass die Winkelprofile (1) im Abstand von den unmittel- bar miteinander verbundenen Enden, vorzugs- weise nahe an oder nahe ihrem jeweils anderen Ende, durch Abstandhalter verbindbar sind, die vorzugsweise aus Metallketten (7) gebildet sind.

4.    Gestell nach einem der vorhergehenden Ansprüche, dadurch gekennzeichnet, dass die Winkelprofile (1) mit einer quadratischen Basisplatte (8), z.B. aus Kupferblech verbindbar sind.

5.   Gestell nach einem der vorhergehenden Ansprüche, dadurch gekennzeichnet, dass an einem der Winkelprofile (1a) ein, seitlich oder von oben ablesbarer Kompass (9) montiert ist, vorzugsweise an jenem Winkelprofil (1a), das bei vollständig zusammengeklapptem Gestell (2) im Inneren der durch die vier Winkelprofile (1) gebildeten Röhre liegt.

6.   Gestell nach einem der vorhergehenden Ansprüche, dadurch gekennzeichnet, dass die Länge eines Winkelprofils (1) etwa um den Faktor 1,2 bis 1,3 vorzugsweise 1,28 grösser ist als die Basislänge zwischen zwei benachbarten Winkelprofilen an deren freien Enden bei vollständig pyramidenförmig aufgespreizten Winkelprofilen (1).

7.   Gestell nach einem der vorhergehenden Ansprüche, dadurch gekennzeichnet, dass zumindest ein Winkelprofil (1) mit einer Erdungsklemme verbindbar ist.

8.   Gestell nach einem der Ansprüche 3 bis 7, dadurch gekennzeichnet, dass die Länge der Abstandhalter oder deren Anbringungsort an den Winkelprofilen variierbar ist.

9.   Gestell nach einem der vorhergehenden Ansprüche, dadurch gekennzeichnet, dass die jeweils vom benachbarten Winkelprofil (1) verdeckte Seite eines Winkelprofils (1) im Bereich der Spitze (5) verlaufend abgerundet ist, und/oder dass die Verbindungsmittel in Abhängigkeit von der Breite der jeweiligen Seite des Winkelprofils (1) um etwa 30% tiefer liegen als eine Ebene parallel zur Basisfläche (8), welche Ebene um eine Breite der jeweiligen Seite unterhalb des Pyramidenspitzes (5) liegt.

10.   Verwendung eines Gestells nach einem der vorhergehenden Ansprüche für das Aktivieren einer Metallfolie durch Pyramidenenergie, und/oder für das Aufnehmen eines Sitz- oder Liegemöbels.

Fig 1

Fig 2

Europäisches
Patentamt

**EUROPÄISCHER RECHERCHENBERICHT**

Nummer der Anmeldung

EP    92 10 5602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | DE-U-8 710 871 (KEPPELER) | | A61N1/16 |
| | * das ganze Dokument * | 1 | |
| | * Seite 8, Zeile 21 - Zeile 26 * | 2 | |
| | * Seite 16, Zeile 26 - Zeile 33 * | 6 | |
| | --- | | |
| A | US-A-1 415 482 (REED) | 1,3,8 | |
| | * das ganze Dokument * | | |
| | --- | | |
| A | FR-A-2 506 533 (CORNILLON) | 1,4,10 | |
| | * das ganze Dokument * | | |
| | --- | | |
| A | GB-A-385 987 (STETTNER) | 1,5,10 | |
| | * das ganze Dokument * | | |
| | --- | | |
| A | GB-A-2 227 931 (COGHILL) | 1,7,8,10 | |
| | * das ganze Dokument * | | |
| | --- | | |
| A | DE-C-819 714 (ANDRAULT) | 1,9 | |
| | * das ganze Dokument * | | |
| | | | **RECHERCHIERTE SACHGEBIETE (Int. Cl.5 )** |
| | ----- | | |
| | | | A61N |
| | | | E04H |
| | | | E04B |

Der vorliegende Recherchenbericht wurde für alle Patentansprüche erstellt

| Recherchenort | Abschlußdatum der Recherche | Prüfer |
|---|---|---|
| DEN HAAG | 16 JULI 1992 | LEMERCIER D.L.L. |